(19) Europäisches Patentamt
European Patent Office
Office européen des brevets

(11) Numéro de publication: **0 432 006 B1**

(12) # FASCICULE DE BREVET EUROPEEN

(45) Date de publication de fascicule du brevet: **05.10.94**

(51) Int. Cl.$^5$: **C07C 37/60**

(21) Numéro de dépôt: **90403318.0**

(22) Date de dépôt: **23.11.90**

(54) **Procédé d'hydroxylation de phénols et d'éthers de phénols.**

(30) Priorité: **05.12.89 FR 8916312**

(43) Date de publication de la demande:
**12.06.91 Bulletin 91/24**

(45) Mention de la délivrance du brevet:
**05.10.94 Bulletin 94/40**

(84) Etats contractants désignés:
**AT BE CH DE DK ES FR GB GR IT LI LU NL SE**

(56) Documents cités:
**FR-A- 2 148 253**
**FR-A- 2 211 434**
**FR-A- 2 318 851**

(73) Titulaire: **RHONE-POULENC CHIMIE**
**25, quai Paul Doumer**
**F-92408 Courbevoie Cédex (FR)**

(72) Inventeur: **Costantini, Michel**
**10, rue du Docteur Bonhomme**
**F-69003 Lyon (FR)**
Inventeur: **Laucher, Dominique**
**205, Avenue Jean-Jaurès**
**F-69007 Lyon (FR)**

(74) Mandataire: **Dutruc-Rosset, Marie-Claude et al**
**RHONE-POULENC INTERSERVICES**
**Service Brevets Chimie**
**25, Ouai Paul Doumer**
**F-92408 Courbevoie Cédex (FR)**

**EP 0 432 006 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services
(3.10/3.09/3.3.3)

## Description

La présente invention concerne l'hydroxylation de phénols et d'éthers de phénols par le peroxyde d'hydrogène.

Le brevet français n° 69.45467 publié sous le numéro 2 071 464 décrit un procédé d'hydroxylation de phénols et d'éthers de phénols par l'eau oxygénée, en présence d'un acide fort. Parmi ces acides forts l'acide sulfurique, l'acide paratoluènesulfonique, l'acide perchlorique sont les plus utilisés.

Le procédé selon ce brevet est un procédé industriel très important. Cependant la littérature fait état depuis quelques années de la recherche d'une catalyse de cette réaction, qui serait moins corrosive pour l'appareillage et conduirait à des rendements au moins aussi bons.

Ainsi la demande de brevet français publiée sous le numéro FR 2 489 816 préconise l'utilisation de silicalites de différents métaux.

La demande de brevet européen EP-A-0 299 893 décrit l'utilisation d'argiles pontées.

Bien que ces procédés utilisant une catalyse hétérogène paraissent intéressants, il semble qu'il se pose encore des problèmes industriels tels que le recyclage du catalyseur, sa régénération et son vieillissement.

La présente invention permet d'obtenir des rendements d'hydroxylation plus élevés qu'avec les acides forts.

Plus précisément l'invention consiste en un procédé d'hydroxylation de phénols ou d'éthers de phénols de formule générale (I) :

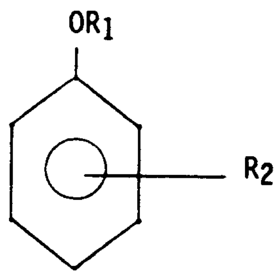

(I)

dans laquelle :

- $R_1$ représente un atome d'hydrogène, un groupement méthyle, un goupement éthyle ou un groupement phényle ;
- $R_2$ représente un atome d'hydrogène, un radical alkyle ayant de 1 à 4 atomes de carbone, un radical alcoxy ayant de 1 à 4 atomes de carbone, un radical phényle ou cyclohexyle,

par réaction avec le peroxyde d'hydrogène, caractérisé en ce que l'on opère en présence :

- d'une quantité efficace d'au moins un sel de métal alcalin ou alcalino-terreux d'un acide protonique ayant un pKa dans l'eau inférieur à - 0,1 ,
- et d'une quantité efficace de l'acide protonique libre.

Les acides protoniques servant de catalyseurs dans le présent procédé sont de préférence ceux dont le pKa dans l'eau est inférieur à - 1.

Le pKa est défini comme la constante de dissociation ionique du couple acide/base, lorsque l'eau est utilisée comme solvant.

Parmi les acides répondant à cette définition, il est préférable d'utiliser ceux qui sont stables vis-à-vis de l'oxydation par le peroxyde d'hydrogène.

On peut citer plus particulièrement l'acide sulfurique, l'acide pyrosulfurique, l'acide perchlorique, l'acide nitrique, les acides halogénosulfoniques tels que l'acide fluorosulfonique, l'acide chlorosulfonique ou l'acide trifluorométhanesulfonique, l'acide méthanesulfonique, l'acide éthanesulfonique, l'acide éthanedisulfonique, l'acide benzènesulfonique, les acides benzènedisulfoniques, les acides toluènesulfoniques, les acides naphtalènesulfoniques et les acides naphtalènedisulfoniques.

Parmi ces acides, on utilisera de préférence l'acide sulfurique, l'acide perchlorique, l'acide trifluorométhanesulfonique, l'acide paratoluènesulfonique, l'acide chlorosulfonique, l'acide fluorosulfonique, l'acide méthanesulfonique.

Les sels de métaux alcalins ou alcalino-terreux d'acide protonique tel que défini précédemment, que l'on utilise conjointement avec un acide protonique libre dans le présent procédé, sont plus particulièrement les sels de métaux alcalins ou alcalino-terreux de l'acide sulfurique, de l'acide pyrosulfurique, de l'acide perchlorique, de l'acide nitrique, des acides halogénosulfoniques tels que l'acide chlorosulfonique, l'acide

fluorosulfonique ou l'acide trifluorométhanesulfonique, de l'acide méthanesulfonique, de l'acide éthanesulfonique, de l'acide éthanedisulfonique, de l'acide benzènesulfonique, des acides benzènedisulfoniques, des acides toluènesulfoniques, des acides naphtalènesulfoniques et des acides naphtalènedisulfoniques.

Par sels de métaux alcalins, on entend dans le présent texte les sels neutres des acides définis précédemment de lithium, de sodium, de potassium, de rubidium et de césium.

On préfère le plus souvent utiliser les sels de sodium ou de potassium et encore plus préférentiellement, pour des raisons économiques, les sels de sodium.

Parmi ces différents sels, on peut citer parmi les préférés le sulfate disodique, le perchlorate de sodium, le trifluorométhanesulfonate de sodium, le paratoluènesulfonate de sodium, le chlorosulfonate de sodium, le fluorosulfonate de sodium, le méthanesulfonate de sodium.

Par les sels de métaux alcalino-terreux, on entend dans le présent texte les sels neutres des acides définis précédemment de beryllium, de magnésium, de calcium, de strontium et de baryum.

On préfère le plus souvent utiliser les sels de magnésium, de calcium et de baryum.

Parmi ces différents sels de métaux alcalino-terreux, on mettra en oeuvre préférentiellement le sulfate de calcium, le sulfate de magnésium, le perchlorate de calcium, le perchlorate de magnésium, le trifluorométhanesulfonate de calcium, le trifluorométhanesulfonate de magnésium, le paratoluènesulfonate de calcium, le paratoluènesulfonate de magnésium, le fluorosulfonate de calcium, le fluorosulfonate de magnésium, le méthanesulfonate de calcium, le méthanesulfonate de magnésium.

On peut utiliser des mélanges de plusieurs sels de métaux alcalins ou alcalino-terreux d'un même acide protonique.

La quantité globale d'acide protonique et de sel de métal alcalin ou alcalino-terreux peut varier dans de larges limites.

Généralement cette quantité est exprimée en rapport molaire acide protonique + sel de métal alcalin ou alcalino-terreux/peroxyde d'hydrogène.

Ce rapport est le plus souvent compris entre 0,1 % et 25 % en moles et de préférence entre 0,5 % et 15 % en moles.

Le rapport molaire sel de métal alcalin ou alcalino-terreux/acide protonique libre est généralement compris entre 0,2 et 20 et de préférence entre 0,5 et 10.

Le sel de métal alcalin ou alcalino-terreux peut être préparé in situ, par exemple en chargeant l'acide protonique que l'on souhaite mettre en oeuvre et un oxyde ou un hydroxyde de métal alcalin ou alcalino-terreux en quantités telles qu'il y ait un excès d'acide par rapport à la quantité stoechiométrique.

Cet excès sera calculé de telle sorte que le rapport molaire sel de métal alcalin ou alcalino-terreux/acide protonique libre soit dans la zone de valeurs indiquée précédemment.

On peut utiliser avantageusement, avec les catalyseurs constitués par les acides protoniques et leurs sels de métaux alcalins ou alcalino-terreux, un oxacide du phosphore qui améliore le rendement en diphénols.

Les oxacides du phosphore sont plus particulièrement les composés à fonction acide du phosphore au degré d'oxydation 5.

On peut également utiliser des composés à fonction acide du phosphore au degré d'oxydation 3, qui seront oxydés dans le milieu par le peroxyde d'hydrogène en composés correspondants du phosphore V ; mais cela ne présente pas d'intérêt particulier, tout en ayant l'inconvénient de consommer une partie du peroxyde d'hydrogène.

Parmi ces oxacides du phosphore V, on peut citer par exemple l'acide orthophosphorique, l'acide métaphosphorique, l'acide pyrophosphorique, les acides polyphosphoriques, les acides phosphoniques tels que l'acide (hydroxy-1 éthylidène)diphosphonique, l'acide phosphonique, l'acide éthylphosphonique, l'acide phénylphosphonique. Parmi ces acides, on utilisera de préférence l'acide orthophosphorique, l'acide pyrophosphorique et l'acide (hydroxy-1 éthylidène)diphosphonique.

La quantité d'oxacide du phosphore exprimée en rapport molaire oxacide du phosphore/peroxyde d'hydrogène est comprise entre 0,05 % et 20 % et de préférence entre 0,1 et 10 %.

Le peroxyde d'hydrogène peut être mis en oeuvre sous forme de solution aqueuse ou de solution organique.

Les solutions aqueuses étant commercialement plus facilement disponibles sont utilisées de préférence. Généralement elles contiennent plus de 20 % en poids de peroxyde d'hydrogène.

La quantité de peroxyde d'hydrogène peut aller jusqu'à 1 mole de $H_2O_2$ pour 1 mole de composé phénolique de formule (I).

Il est cependant préférable pour obtenir des rendements industriellement acceptables d'utiliser un rapport molaire composé phénolique de formule (I)/peroxyde d'hydrogène de 25/1 à 3/1 et de préférence de 20/1 à 4/1.

Parmi les composés phénoliques de formule (I) qui pourront être mis en oeuvre dans le procédé de l'invention, on peut citer à titre non limitatif le phénol, l'anisole, l'orthocrésol, le paracrésol, le métacrésol, le tertiobutyl-4 phénol, le méthoxy-2 phénol, le méthoxy-4 phénol.

Le présent procédé convient tout particulièrement bien à la préparation d'hydroquinone et de pyrocatéchol à partir du phénol.

Le procédé d'hydroxylation est généralement mis en oeuvre sans solvant autre que celui qui provient des réactifs, comme le solvant du peroxyde d'hydrogène.

La réaction peut cependant également être réalisée dans un solvant du composé phénolique (I).

Les solvants éventuellement utilisés sont nombreux.

On peut citer des solvants non polaires comme les hydrocarbures aliphatiques chlorés, par exemple le dichlorométhane, le tétrachlorométhane, le dichloroéthane.

On peut également citer des solvants peu polaires comme les alcools et les éthers, par exemple le méthanol, le tertiobutanol, l'isopropanol, l'éthanol, le méthyl-tertiobutyléther ou très polaires comme l'eau.

En règle générale, les solvants non polaires sont plus favorables et sont utilisés de préférence.

La température à laquelle est conduite la réaction d'hydroxylation est généralement comprise entre 45 °C et 160 °C sous pression atmosphérique.

On peut également opérer à des températures plus élevées sous une pression supérieure à la pression atmosphérique.

Les réactifs et les conditions opératoires conviennent bien à une mise en oeuvre en continu du procédé de l'invention.

Les exemples qui suivent illustrent l'invention.

EXEMPLES 1 A 6 ET ESSAIS A, B ET C

Dans un ballon en verre de 100 $cm^3$, muni d'un réfrigérant ascendant, d'un thermomètre, d'une ampoule de coulée et d'une agitation centrale, on charge :

- phénol : 47 g(0,5 mol)
- perchlorate : nature et rapport molaire avec $H_2O_2$ indiquésdans le tableau 1
- acide perchlorique : rapport molaire avec $H_2O_2$ indiqué dans le tableau 1
- acide pyrophosphorique : rapport molaire avec $H_2O_2$ indiquédans le tableau I.

Après avoir porté ce mélange à 75 °C sous agitation, on introduit une solution aqueuse de peroxyde d'hydrogène en quantité telle que l'on ait un rapport molaire $H_2O_2$/phénol de 10 % ou de 5 % selon les exemples.

Après chauffage à 75 °C pendant les durées indiquées dans le tableau 1, l'essai est refroidi, le peroxyde d'hydrogène éventuellement restant est dosé par iodométrie et les diphénols formés sont dosés par chromatographie liquide haute performance (CLHP).

Le tableau 1 ci-après rassemble les résultats obtenus.

Les abréviations suivantes sont utilisées dans ce tableau :

TT % : taux de transformation de $H_2O_2$ en %
RT % HQ : rendement % en hydroquinone par rapport à $H_2O_2$ transformé
RT % PC : rendement % en pyrocatéchol par rapport à $H_2O_2$ transformé
RT % total : rendement % total en diphénols par rapport à $H_2O_2$ transformé.

On note que le rendement total en diphénols est plus élevé lorsque l'on catalyse la réaction d'hydroxylation par l'acide perchlorique et un perchlorate métallique que lorsque la catalyse est effectuée avec l'acide perchlorique seul.

La comparaison de l'exemple 1 avec l'essai A (pour le même rapport $ClO_4^-/H_2O_2$) montre que le rendement est plus élevé avec le mélange acide perchlorique/perchlorate de magnésium qu'avec le seul acide perchlorique.

TABLEAU 1

| ESSAIS | $H_2O_2$/phénol % en moles | $HClO_4/H_2O_2$ % en moles | Sel | | $H_4P_2O_7/H_2O_2$ % en moles | Durée | TT % $H_2O_2$ | RT % HQ | RT % PC | RT % total | PC/HQ |
|---|---|---|---|---|---|---|---|---|---|---|---|
| | | | Nature | Sel/$H_2O_2$ % en moles | | | | | | | |
| Exemple 1 | 10 | 1,2 | $Mg(ClO_4)_2$ | 3,6 | 0,6 | 1 h | 99,5 | 32,0 | 45,0 | 77,0 | 1,4 |
| Essai comparatif A | 10 | 8,4 | néant | 0 | 0,6 | 0 h 50 | 100 | 13,0 | 31,5 | 44,5 | 2,4 |
| Exemple 2 | 10 | 1,2 | $Mg(ClO_4)_2$ | 7,9 | 0,6 | 2 h 25 | 100 | 31,5 | 44,0 | 75,5 | 1,4 |
| Exemple 3 | 10 | 1,3 | $NaClO_4,H_2O$ | 3,6 | 0,6 | 2 h 30 | 99,5 | 28,5 | 45,0 | 73,5 | 1,6 |
| Exemple 4 | 10 | 1,3 | $NaClO_4,H_2O$ | 7,3 | 0,6 | 1 h | 99 | 29,5 | 44,0 | 73,5 | 1,5 |
| Essai comparatif B | 10 | 1,3 | néant | 0 | 0,6 | 2 h 25 | 100 | 30,0 | 42,0 | 72,0 | 1,4 |
| Exemple 5 | 5 | 1,4 | $Mg(ClO_4)_2$ | 1,2 | 0,6 | 0 h 40 | 100 | 37,0 | 51,0 | 88,0 | 1,4 |
| Exemple 6 | 5 | 2,3 | $Mg(ClO_4)_2$ | 14 | 1,3 | 0 h 30 | 100 | 38,0 | 49,5 | 87,5 | 1,3 |
| Essai comparatif C | 5 | 1,4 | néant | 0 | 0,6 | 1 h | 100 | 35,0 | 49,0 | 84,0 | 1,4 |

EXEMPLES 7 A 14 ET ESSAIS COMPARATIFS D ET E

On répète les exemples 1 à 6 en utilisant comme catalyseurs l'acide sulfurique et un sulfate de sodium ou de magnésium.

Tous les essais sont réalisés avec 0,6 % en moles de $H_4P_2O_7$ par rapport à $H_2O_2$.

Le tableau 2 ci-après indique la nature des différents catalyseurs, leur quantité (en % molaire par rapport à $H_2O_2$), ainsi que celle de $H_2O_2$ (en % molaire par rapport au phénol), la température, la durée et les résultats obtenus.

Les abréviations utilisées sont les mêmes que pour le tableau 1.

TABLEAU 2

| ESSAIS | $H_2O_2$/phénol % en moles | $H_2SO_4/H_2O_2$ % en moles | Sel | | Température °C | Durée | TT % $H_2O_2$ | RT % HQ | RT % PC | RT % total | PC/HQ |
|---|---|---|---|---|---|---|---|---|---|---|---|
| | | | Nature | Sel/$H_2O_2$ % en moles | | | | | | | |
| Exemple 7 | 5,5 | 1,1 | $Na_2SO_4,H_2O$ | 2,7 | 75 | 6 h 40 | 96,5 | 27,0 | 54,5 | 81,5 | 2,0 |
| Exemple 8 | 5 | 1,7 | $Na_2SO_4,H_2O$ | 3,7 | 75 | 1 h 45 | 100 | 28,5 | 55,5 | 84,0 | 1,95 |
| Exemple 9 | 6 | 1,5 | $Na_2SO_4,H_2O$ | 5,2 | 75 | 4 h 30 | 100 | 28,5 | 57,0 | 85,5 | 2,0 |
| Exemple 10 | 5 | 1,5 | $MgSO_4,7H_2O$ | 3,7 | 100 | 1 h | 100 | 28,5 | 56,0 | 84,5 | 1,95 |
| Exemple 11 | 5 | 2,0 | $MgSO_4$ | 3,0 | 100 | 1 h | 100 | 29,0 | 55,5 | 84,5 | 2,0 |
| Exemple 12 | 5 | 1,2 | $MgSO_4$ | 3,6 | 100 | 1 h | 100 | 28,5 | 57,0 | 85,5 | 2,0 |
| Exemple 13 | 5 | 1,8 | $MgSO_4$ | 4,2 | 100 | 1 h | 100 | 28,5 | 56,0 | 84,5 | 2,0 |
| Exemple 14 | 10 | 1,4 | $MgSO_4$ | 3,7 | 100 | 1 h | 100 | 25,5 | 50,0 | 75,5 | 1,95 |
| Essai comparatif D | 5 | 1,4 | néant | 0 | 75 | 4 h 30 | 100 | 27,0 | 53,0 | 80,0 | 1,95 |
| Essai comparatif E | 5,5 | 1,4 | néant | 0 | 100 | 1 h | 100 | 27,0 | 52,5 | 79,5 | 1,95 |

EXEMPLES 15 A 18 ET ESSAIS F, G ET H : essais en milieu solvant

On répète les exemples 1 à 6 en opérant en présence d'un solvant : soit dichlorométhane
soit eau

Les essais sont effectués avec 0,3 % en moles de $H_4P_2O_7/H_2O_2$.

Le tableau 3 ci-après indique la nature des catalyseurs, leur quantité (en % molaire par rapport à $H_2O_2$), la quantité de $H_2O_2$ (en % molaire par rapport au phénol), la quantité de solvant, la température, la durée et les résultats obtenus.

Les abréviations utilisées sont les mêmes que dans le tableau 1.

TABLEAU 3

| ESSAIS | $H_2O_2$/phénol % en moles | Acide/$H_2O_2$ % en moles | Sel | | Solvant poids | Température °C | Durée | TT % $H_2O_2$ | RT % HQ | RT % PC | RT % total | PC/HQ |
|---|---|---|---|---|---|---|---|---|---|---|---|---|
| | | | Nature | Sel/$H_2O_2$ % en moles | | | | | | | | |
| Exemple 15 | 5,5 | $H_2SO_4$ 1,5 | $MgSO_4$ | 3,2 | $CH_2Cl_2$ 12,8 g | 75 | 3 h 30 | 100 | 24,5 | 56,5 | 81,0 | 2,3 |
| Essai comparatif F | 5,5 | $H_2SO_4$ 1,7 | néant | 0 | $CH_2Cl_2$ 12,8 g | 75 | 3 h 30 | 100 | 22,0 | 53,0 | 75,0 | 2,4 |
| Exemple 16 | 5,5 | $H_2SO_4$ 1,5 | $MgSO_4$ | 3,8 | $H_2O$ 2,7 g | 100 | 3 h 10 | 100 | 23,0 | 47,0 | 70,0 | 2,0 |
| Exemple 17 | 5,5 | $H_2SO_4$ 1,5 | $MgSO_4$ | 10,1 | $H_2O$ 2,7 g | 100 | 3 h | 100 | 22,5 | 48,5 | 71,0 | 2,2 |
| Essai comparatif G | 5,5 | $H_2SO_4$ 1,9 | néant | 0 | $H_2O$ 2,7 g | 100 | 1 h 30 | 99,5 | 10,0 | 33,5 | 43,5 | 3,3 |
| Exemple 18 | 5,0 | $HClO_4$ 1,3 | $Mg(ClO_4)_2$ | 3,6 | $CH_2Cl_2$ 12,8 g | 75 | 2 h | 100 | 33,5 | 49,5 | 83,0 | 1,5 |
| Essai comparatif H | 5,5 | $HClO_4$ 1,3 | néant | 0 | $CH_2Cl_2$ 12,8 g | 75 | 2 h 10 | 100 | 32,0 | 48,5 | 80,5 | 1,5 |

## Revendications

**1.** Procédé d'hydroxylation de phénols ou d'éthers de phénols de formule générale (I) :

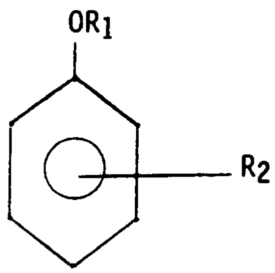

(I)

dans laquelle :

- $R_1$ représente un atome d'hydrogène, un groupement méthyle, un groupement éthyle ou un groupement phényle ;
- $R_2$ représente un atome d'hydrogène, un radical alkyle ayant de 1 à 4 atomes de carbone, un radical alcoxy ayant de 1 à 4 atomes de carbone, un radical phényle ou cyclohexyle,

par réaction avec le peroxyde d'hydrogène, caractérisé en ce que l'on opère en présence :

- d'une quantité efficace d'au moins un sel de métal alcalin ou alcalino-terreux d'un acide protonique ayant un pKa dans l'eau inférieur à - 0,1 ;
- et d'une quantité efficace de l'acide protonique libre.

**2.** Procédé selon la revendication 1, caractérisé en ce que les acides protoniques servant de catalyseurs sont ceux dont le pKa dans l'eau est inférieur à - 1.

**3.** Procédé selon l'une des revendications 1 ou 2, caractérisé en ce que l'on utilise l'acide sulfurique, l'acide pyrosulfurique, l'acide perchlorique, l'acide nitrique, les acides halogénosulfoniques tels que l'acide chlorosulfonique, l'acide fluorosulfonique ou l'acide trifluorométhanesulfonique, l'acide méthanesulfonique, l'acide éthanesulfonique, l'acide éthanedisulfonique, l'acide benzènesulfonique, les acides benzènedisulfoniques, les acides toluènesulfoniques, les acides naphtalènesulfoniques et les acides naphtalènedisulfoniques avec leurs sels respectifs de métaux alcalins et alcalino-terreux.

**4.** Procédé selon l'une des revendications 1 à 3, caractérisé en ce que l'on utlise comme sels de métaux alcalins, les sels neutres des acides définis dans la revendication 3 de lithium, de sodium, de potassium, de rubidium et de césium et de préférence les sels de sodium ou de potassium.

**5.** Procédé selon l'une des revendications 1 à 4, caractérisé en ce que l'on utilise comme sels de métaux alcalins le sulfate disodique, le perchlorate de sodium, le trifluorométhanesulfonate de sodium, le paratoluènesulfonate de sodium, le chlorosulfonate de sodium, le fluorosulfonate de sodium, le méthanesulfonate de sodium.

**6.** Procédé selon l'une des revendications 1 à 3, caractérisé en' ce que l'on utilise comme sels de métaux alcalino-terreux, les sels neutres des acides définis dans la revendication 3 de beryllium, de magnésium, de calcium, de strontium et de baryum et de préférence les sels de magnésium, de calcium et de baryum.

**7.** Procédé selon l'une des revendications 1 à 3 et 6, caractérisé en ce que l'on utilise comme sels de métaux alcalinoterreux le sulfate de calcium, le sulfate de magnésium, le perchlorate de calcium, le perchlorate de magnésium, le trifluorométhanesulfonate de calcium, le trifluorométhanesulfonate de magnésium, le paratoluènesulfonate de calcium, le paratoluènesulfonate de magnésium, le fluorosulfonate de calcium, le fluorosulfonate de magnésium, le méthanesulfonate de calcium, le méthanesulfonate de magnésium.

**8.** Procédé selon l'une des revendications 1 à 7, caractérisé en ce que l'on opère en présence d'un oxacide du phosphore.

9. Procédé selon la revendication 8, caractérisé en ce que l'oxacide du phosphore est un composé à fonction acide du phosphore au degré d'oxydation 5.

10. Procédé selon la revendication 9, caractérisé en ce que les oxacides du phosphore V sont l'acide orthophosphorique, l'acide métaphosphorique, l'acide pyrophosphorique, les acides polyphosphoriques, les acides phosphoniques tels que l'acide (hydroxy-1 éthylidène) diphosphonique, l'acide phosphonique, l'acide éthylphosphonique, l'acide phénylphosphonique.

11. Procédé selon l'une des revendications 9 ou 10, caractérisé en ce que les oxacides du phosphore sont l'acide orthophosphorique, l'acide pyrophosphorique et l'acide (hydroxy-1 éthylidène) diphosphonique.

12. Procédé selon l'une des revendications 1 à 11, caractérisé en ce que la quantité globale d'acide protonique et de sel de métal alcalin ou alcalino-terreux, exprimée en rapport molaire acide protonique + sel de métal alcalin ou alcalino-terreux/peroxyde d'hydrogène est compris entre 0,1 % et 25 % et de préférence entre 0,5 et 15 %.

13. Procédé selon l'une des revendications 1 à 11, caractérisé en ce que le rapport molaire sel de métal alcalin ou alcalino-terreux/acide protonique est compris entre 0,2 et 20 et de préférence entre 0,5 et 10.

14. Procédé selon l'une des revendications 1 à 11, caractérisé en ce que la quantité d'oxacide du phosphore exprimée en rapport molaire oxacide du phosphore/peroxyde d'hydrogène est comprise entre 0,05 % et 20 % et de préférence entre 0,1 % et 10 %.

15. Procédé selon l'une des revendications 1 à 14, caractérisé en ce que l'on utilise un rapport molaire composé phénolique de formule (I)/peroxyde d'hydrogène de 25/1 à 3/1 et de préférence de 20/1 à 4/1.

16. Procédé selon l'une des revendications 1 à 15, caractérisé en ce que l'on utilise comme composés phénoliques de formule (I) le phénol, l'anisole, l'orthocrésol, le paracrésol, le métacrésol, le tertiobutyl 4 phénol, le méthoxy-2 phénol, le méthoxy-4 phénol.

17. Procédé selon l'une des revendications 1 à 16, caractérisé en ce que la température à laquelle est conduite la réaction d'hydroxylation est comprise entre 45 °C et 160 °C sous pression atmosphérique.

18. Procédé selon l'une des revendications 1 à 17, caractérisé en ce que l'on opère dans un solvant choisi parmi les hydrocarbures aliphatiques chlorés.

**Claims**

1. A method of hydroxylating phenols or ethers of phenols of general formula (I):

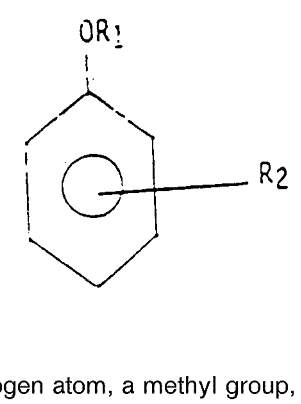

(I)

wherein:

- $R_1$ represents a hydrogen atom, a methyl group, an ethyl group or a phenyl group;
- $R_2$ represents a hydrogen atom, an alkyl radical with 1 to 4 carbon atoms, an alkoxy radical with 1 to 4 carbon atoms or a phenyl or cyclohexyl radical,

by reacting them with hydrogen peroxide, characterised in that the reaction takes place in the presence of:

- an effective quantity of at least one alkali metal or alkaline earth metal salt of a protonic acid with a pKa in water of less than -0.1
- and an effective quantity of free protonic acid.

**2.** The method of Claim 1, characterised in that the protonic acids acting as catalysts are those which have a pKa in water of less than -1.

**3.** The method of Claim 1 or 2, characterised in that sulphuric acid, pyrosulphuric acid, perchloric acid, nitric acid, halosulphonic acids such as chlorosulphonic acid, fluorosulphonic acid or trifluoromethane sulphonic acid, methane sulphonic acid, ethane sulphonic acid, ethane disulphonic acid, benzene sulphonic acid, benzene disulphonic acids, toluene sulphonic acids, naphthalene sulphonic acids and naphthalene disulphonic acids are used with their respective alkali metal and alkaline earth metal salts.

**4.** The method of any of Claims 1 to 3, characterized in that the alkali metal salts are neutral salts of the acids defined in Claim 3 of lithium, sodium, potassium, rubidium and cesium, and preferably salts of sodium or potassium.

**5.** The method of any of Claims 1 to 4, characterized in that the alkali metal salts used are disodium sulphate, sodium perchlorate, sodium trifluoromethane sulphonate, sodium paratoluene sulphonate, sodium chlorosulphonate, sodium fluorosulphonate or sodium methane sulphonate.

**6.** The method of any of Claims 1 to 3, characterized in that the alkaline earth metal salts used are neutral salts of the acids defined in Claim 3 of beryllium, magnesium, calcium, strontium and barium and preferably salts of magnesium, calcium and barium.

**7.** The method of any of Claims 1 to 3 and 6, characterised in that the alkaline earth metal salts used are calcium sulphate, magnesium sulphate, calcium perchlorate, magnesium perchlorate, calcium trifluoromethane sulphonate, magnesium trifluoromethane sulphonate, calcium paratoluene sulphonate, magnesium paratoluene sulphonate, calcium fluorosulphonate, magnesium fluorosulphonate, calcium methane sulphonate or magnesium methane sulphonate.

**8.** The method of any of Claims 1 to 7, characterised in that the operation takes place in the presence of an oxacid of phosphorus.

**9.** The method of Claim 8, characterised in that the oxacid of phosphorus is an acid function compound of phosphorus with level 5 of oxidation.

**10.** The method of Claim 9, characterised in that the oxacids of phosphorus V are orthophosphoric acid, metaphosphoric acid, pyrophosphoric acid, polyphosphoric acids, and phosphonic acids such as (1-hydroxy ethylidene)disphosphonic acid, phosphonic acid, ethyl phosphonic acid and phenyl phosphonic acid.

**11.** The method of Claim 9 or 10, characterized in that the oxacids of phosphorus are orthosphosphoric acid, pyrophosphoric acid and (1-hydroxy ethylidene)disphosphonic acid.

**12.** The method of any of Claims 1 to 11, characterised in that the total quantity of protonic acid and of alkali metal or alkaline earth metal salt, expressed as a molar ratio of protonic acid plus alkali metal or alkaline earth metal salt to hydrogen peroxide, is from 0.1 to 25% and preferably from 0.5 to 15%.

**13.** The method of any of Claims 1 to 11, characterised in that the molar ratio of alkali metal or alkaline earth metal salt to protonic acid is from 0.2 to 20 and preferably from 0.5 to 10.

**14.** The method of any of Claims 1 to 11, characterised in that the quantity of phosphorus oxacid expressed as a molar ratio of phosphorus oxacid to hydrogen peroxide is from 0.05 to 20% and preferably from 0.1 to 10%.

**15.** The method of any of Claims 1 to 14, characterized in that the molar ratio of formula (I) phenolic compound to hydrogen peroxide used is from 25:1 to 3:1 and preferably from 20:1 to 4:1.

16. The method of any of Claims 1 to 15, characterized in that the formula (I) phenolic compounds used are phenol, anisol, orthocresol, paracresol, metacresol, 4-tertiobutyl phenol, 2-methoxy phenol or 4-methoxy phenol.

17. The method of any of Claims 1 to 16, characterized in that the temperature at which the hydroxylation reaction is carried out is from 45 to 160 ° C at atmospheric pressure.

18. The method of any of Claims 1 to 17, characterised in that the operation is carried out in a solvent selected from chlorinated aliphatic hydrocarbons.

**Patentansprüche**

1. Verfahren zur Hydroxylierung von Phenolen oder Phenolethern der allgemeinen Formel (I):

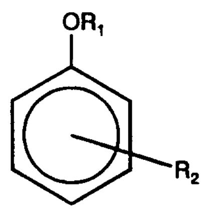

(I)

in der:
- $R_1$ ein Wasserstoffatom, eine Methyl- Ethyl- oder Phenylgruppe darstellt;
- $R_2$ ein Wasserstoffatom, einen Alkylrest mit 1 bis 4 Kohlenstoffatomen, einen Alkoxyrest mit 1 bis 4 Kohlenstoffatomen, einen Phenyl- oder Cyclohexylrest darstellt,

durch Reaktion mit Wasserstoffperoxid, dadurch gekennzeichnet, daß die Reaktion in Gegenwart:
- einer wirksamen Menge wenigstens eines Alkali- oder Erdalkalimetallsalzes einer protonischen Säure mit einem pKa in Wasser von weniger als -0,1;
- und einer wirksamen Menge der freien protonischen Säure

durchgeführt wird.

2. Verfahren nach Anspruch 1, dadurch gekennzeichnet, daß die protonischen Säuren, die als Katalysator dienen, einen pKa in Wasser von weniger als -1 besitzen.

3. Verfahren nach Anspruch 1 oder 2, dadurch gekennzeichnet, daß Schwefelsäure, Pyroschwefelsäure, Perchlorsäure, Salpetersäure, Halogensulfonsäuren, wie Chlorsulfonsäure, Fluorsulfonsäure oder Trifluormethansulfonsäure, Methansulfonsäure, Ethansulfonsäure, Ethandisulfonsäure, Benzolsulfonsäure, Benzoldisulfonsäuren, Toluolsulfonsäuren, Naphthalinsulfonsäuren und die Naphthalindisulfonsäuren sowie deren entsprechende Salze von Alkali- und Erdalkalimetallen verwendet werden.

4. Verfahren nach einem der Ansprüche 1 bis 3, dadurch gekennzeichnet, daß als Alkalimetallsalze die neutralen Lithium-, Natrium-, Kalium-, Rubidium- und Cäsiumsalze der in Anspruch 3 definierten Säuren und vorzugsweise die Natrium- oder Kaliumsalze verwendet werden.

5. Verfahren nach einem der Ansprüche 1 bis 4, dadurch gekennzeichnet, daß als Alkalimetallsalze Dinatriumsulfat; Natriumperchlorat, Nathumthfluormethansulfonat, Natrium-p-toluolsulfonat, Natriumchlorsulfonat, Natriumfluorsulfonat, Natriummethansulfonat verwendet wird.

6. Verfahren nach einem der Ansprüche 1 bis 3, dadurch gekennzeichnet, daß als Erdalkalimetallsalze die neutralen Beryllium-, Magnesium-, Calcium-, Strontium- und Bariumsalze der in Anspruch 3 definierten Säuren, und vorzugsweise die Magnesium-, Calcium- und Bariumsalze verwendet werden.

7. Verfahren nach einem der Ansprüche 1 bis 3 und 6, dadurch gekennzeichnet, daß als Erdalkalimetallsalze Calciumsulfat, Magnesiumsulfat, Calciumperchlorat, Magnesiumperchlorat, Calciumtrifluormethansulfonat, Magnesiumtrifluormethansulfonat, Calcium-p-toluolsulfonat, Magnesium-p-toluolsulfonat, Calciumfluorsulfonat, Magnesiumfluorsulfonat, Calciummethansulfonat, Magnesiummethansulfonat verwendet

werden.

8. Verfahren nach einem der Ansprüche 1 bis 7, dadurch gekennzeichnet, daß es in Gegenwart einer Oxosäure von Phosphor durchgeführt wird.

9. Verfahren nach Anspruch 8, dadurch gekennzeichnet, daß die Oxosäure von Phosphor eine saure Verbindung des Phosphors mit der Oxidationsstufe 5 ist.

10. Verfahren nach Anspruch 9, dadurch gekennzeichnet, daß die Oxosäuren von Phosphor (V) Orthophosphorsäure, Metaphosphorsäure, Pyrophosphorsäure, Polyphosphorsäuren, Phosphonsäuren, wie 1-Hydroxyethylidendiphosphonsäure, Phosphonsäure, Ethylphosphonsäure, Phenylphosphonsäure sind.

11. Verfahren nach Anspruch 9 oder 10, dadurch gekennzeichnet, daß die Oxosäuren von Phosphor Orthophosphorsäure, Pyrophosphorsäure und 1-Hydroxyethylidendiphosphonsäure sind.

12. Verfahren nach einem der Ansprüche 1 bis 11, dadurch gekennzeichnet, daß die Gesamtmenge der protonischen Säure und des Alkali- oder Erdalkalimetallsalzes, ausgedrückt als Molverhältnis protonische Säure + Alkali- oder Erdalkalimetallsalz/Wasserstoffperoxid im Bereich von 0,1 bis 25 % und vorzugsweise von 0,5 bis 15 % liegt.

13. Verfahren nach einem der Ansprüche 1 bis 11, dadurch gekennzeichnet, daß das Molverhältnis des Alkali- oder Erdalkalimetallsalzes zur protonischen Säure im Bereich von 0,2 bis 20 und vorzugsweise von 0,5 bis 10 liegt.

14. Verfahren nach einem der Ansprüche 1 bis 11, dadurch gekennzeichnet, daß die Menge der Oxosäure von Phosphor, ausgedrückt als Molverhältnis Oxosäure von Phosphor/Wasserstoffperoxid im Bereich von 0,05 bis 20 % und vorzugsweise von 0,1 bis 10 % liegt.

15. Verfahren nach einem der Ansprüche 1 bis 14, dadurch gekennzeichnet, daß ein Molverhältnis Phenolverbindung der Formel (I)/Wasserstoffperoxid von 25/1 bis 3/1 und vorzugsweise von 20/1 bis 4/1 verwendet wird.

16. Verfahren nach einem der Ansprüche 1 bis 15, dadurch gekennzeichnet, daß als Phenolverbindungen der Formel (I) Phenol, Anisol, o-Cresol, p-Cresol, m-Cresol, 4-tert.-Butylphenol, 2-Methoxyphenol, 4-Methoxyphenol verwendet werden.

17. Verfahren nach einem der Ansprüche 1 bis 16, dadurch gekennzeichnet, daß die Temperatur, bei der die Hydroxylierungsreaktion durchgeführt wird, im Bereich von 45 bis 160 °C bei Atmosphärendruck liegt.

18. Verfahren nach einem der Ansprüche 1 bis 17, dadurch gekennzeichnet, daß es in einem Lösungsmittel, ausgewählt aus chlorierten aliphatischen Kohlenwasserstoffen durchgeführt wird.